# EUROPEAN PATENT APPLICATION

(11) **EP 1 336 610 A1**
(43) Date of publication of application: **20.08.2003**
(21) Application number: 01997483.1
(22) Date of filing: 21.11.2001
(51) Int. Cl.: C07D 311/72, B01D 15/08, B01D 15/00

(54) **METHOD FOR CHROMATOGRAPHIC PREPARATION OF TOCOTRIENOL**

(30) Priority: 21.11.2000 JP 2000353871
(71) Applicant: Organo Corporation, Tokyo 136-8631 (JP)
(72) Inventor: KANEKO, Kikuzo, Yokohama-shi, Kanagawa 222-0011 (JP); IKUSHIMA, Heiji, FUJI CHEMICAL INDUSTRY CO., LTD., Nakaniikawa-gun, Toyama 930-0397 (JP)
(74) Representative: Viering, Jentschura & Partner
(86) International application number: JP0110164
(87) International publication number: WO02042287

(57) **Abstract**

A simulated moving bed type chromatographic separation apparatus is provided in which a plurality of columns each filled with a hydrophobic adsorbent for reversed-phase liquid chromatography having a relatively low affinity for tocotrienol than that for tocopherol are connected cyclically and in series. To the chromatographic separation apparatus, a raw material fluid containing at least several tens of percent of tocopherol and tocotrienol and consisting substantially 100% of oil constituents and an eluent of ethanol-water = 90/10 are supplied so that a fraction fluid enriched with tocotrienol is separated from tocopherol. With this configuration, preparation of tocotrienol from a raw material fluid containing a large amount of tocopherol and tocotrienol can be achieved in a commercial scale through a method not too different from the conventionally known simulated moving bed type chromatographic separation and the initial investment cost and running cost can be reduced.

## Description

### Technical Field

The present invention relates to a method for commercially, effectively, and inexpensively preparing tocotrienol, and more particularly, to a method for chromatographically preparing tocotrienol wherein the target tocotrienol can be separated and recovered from a raw material fluid containing tocopherol homologues using a simulated moving bed method.

### Background Art

Recently, it has been found that active oxygen, lipid peroxides and the like are significantly associated with adult diseases and aging of human beings. The importance of prevention of oxidation not only within a biological body, but also outside the biological body (such as food) is increasingly recognized. For such prevention of oxidation, biologically active materials such as the tocopherol family (group) having a strong capability of eliminating radicals and a quencher capability and which are commonly called "vitamin E" have attracted attention, and techniques for obtaining the tocopherol family inexpensively, effectively, and in a large amount have been proposed (for example, in Japanese Patent Laid-Open Publication No. Hei 8-59647). The tocopherol family is known to be comprised of 8 homologues, more specifically, α-tocopherol, β-tocopherol, γ-tocopherol, δ-tocopherol, α-tocotrienol, (β-tocotrienol, γ-tocotrienol, and δ-tocotrienol. These homologues do not have completely identical properties, and, for example, the α-tocopherol is known to have a strong biological activity, be effective in a biological body, and be suited for medical purposes and nutritional enhancement.

Among the tocopherol homologues, tocotrienol has been considered as having a weak biological activity, but recent researches revealed that the tocotrienol has a strong oxidation prevention capability. Thus, techniques have been proposed for using the tocotrienol as oxidation preventing agent or the like for medication, food, etc. (for example, refer to Japanese Patent Laid-Open Publication Nos. Hei 8-12532, Hei 8-92050, Hei 8-92062, Hei 9-157136, Hei 9-183995, etc.). In addition, Japanese Patent Laid-Open Publication No. Hei 8-92050 discloses a technique for separating, through a reversed-phase chromatography, each of α-tocotrienol, (β-tocotrienol, γ-tocotrienol, and δ-tocotrienol from a fluid containing tocotrienol groups (α-tocofrienol, (β-tocotrienol, γ-tocotrienol, and δ-tocotrienol) which are separated from tocopherol.

In cases wherein tocopherol and tocotrienol may be used in a mixed state, it is not necessary to purify and separate tocopherol and tocotrienol. However, in order to effectively take advantage of the different properties of the tocotrienol and tocopherol, it is desirable that these materials (tocopherol and tocotrienol) be separated for each use. For example, in the uses proposed in the above-described references, a material in which tocotrienol isolated as such is more desirable than a mixture in which tocopherol and tocotrienol are mixed.

In consideration of the above, a method for separating and recovering (preparing) tocopherol and tocotrienol from a raw material fluid containing a large amount of tocotrienol in a commercially advantageous manner is desired, but no effective method for preparation of tocotrienol in an amount that can be used commercially has been proposed.

For example, in the method for separating α-tocotrienol, (β-tocotrienol, γ-tocotrienol, and δ-tocotrienol into individual tocotrienol through the reversed-phase chromatography disclosed in Japanese Patent Laid-Open Publication No. Hei 8-92050, before the separation, the tocotrienol groups (α-tocotrienol, β-tocotrienol, γ-tocotrienol, and δ-tocotrienol) are separated from a tocopherol group in more strict definition, etc. through distillation. However, although distillation does not cause any difficult problem when the treatment amount for separation and recovery is very small and at a laboratory (research) level, distillation suffers from a problem when a very large amount is to be treated commercially in that its running cost including the cost of the thermal energy is very large. A solvent extraction method may also be considered, but because it is not possible to find a suitable solvent or extraction efficiency. Since no conventional technique is known which can be used for commercial scale purification and separation, a new method must be developed.

The present inventors have proposed, separately from the above-described method, a method disclosed in Japanese Patent Laid-Open Publication No. Hei 8-59647 using a normal phase chromatography as a method for separating and recovering (preparing) tocopherol homologues in a broader sense including tocotrienol. However, this method is targeted to separating tocopherol homologues from other oil constituents and is not suited for further preparing tocotrienol from the tocopherol homologues.

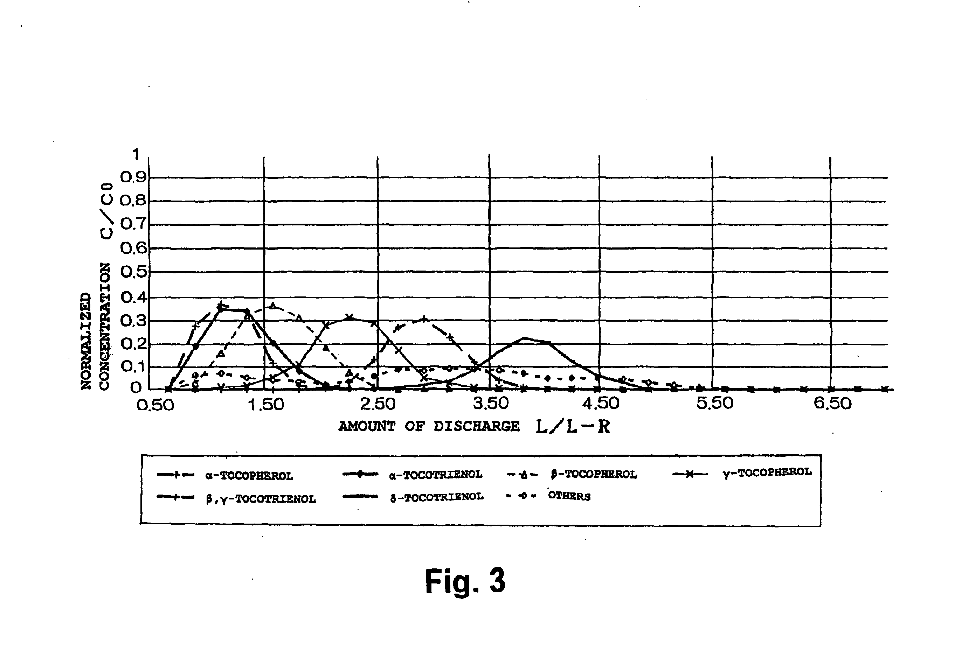

This can be explained as follows. Tocopherol and tocotrienol have similar chemical structures and only differ in that tocopherol has a saturated side chain in a molecule while tocotrienol has three double bonds in a side chain in a molecule. Fig. 3 shows a result of a one-pass experiment of a normal phase chromatography disclosed in Japanese Patent Laid-Open Publication No. Hei 8-59647 which uses an oily eluent having a low polarity which is typically used in chromatographic separation of oil materials. Said experiment was carried out with a chromatographic separation layer of one column filled with an adsorbent according to a preparation method disclosed in Japanese Patent Laid-Open Publication No. Hei 8-92050. As is clear from Fig. 3, the separation bands for α-tocopherol and for α-tocotrienol, which are both present in a relatively large amount are very close, and, therefore, these enriched (rich) bands cannot be sufficiently separated when a simulated moving bed separation operation is performed. As is known to a person with ordinary skill in the art, the suitable relationships among the raw material fluid, the target material to be recovered, the adsorbent, and the eluent are complex in chromatographic separation and it is not easy even for a person with ordinary skill in the art to find the suitable relationships. In addition, because tocotrienol is a thick oily material having no color to yellowish aerial color and tends to be more easily altered by air and light than tocopherol, a method in which tocotrienol can be separated and purified quickly, efficiently, and in a large amount is desired.

A method is known in an analysis operation of a slight amount sample wherein an oil constituent which is insoluble in water and soluble in an organic solvent is separated using a reversed-phase chromatography. It may appear that this method of reversed-phase chromatography used in the analysis of a slight amount sample may be applied for preparation of tocotrienol which is a target of the present invention. However, this method is used in the analysis of the slight amount sample because of a special circumstance that the content of oil constituent is approximately 1% or less in an aqueous sample raw material fluid. Application of this technique for analysis of slight amount sample to a separation and recovery of a raw material fluid containing a large amount of oil constituent by increasing the scale of the method in quantity cannot be accomplished because of the following problem associated with the commercial technology, and, in fact, no such method had been proposed in the conventional art.

Specifically, in an analysis technique for slight amount samples targeted for, for example, research or the like, no importance is given to the economics of the analysis. On the other hand, in a commercial scale application of any such technique, the economical efficiency thereof is very important. For separation and recovery in a commercial scale of tocotrienol as described, a technique is desired for effectively and inexpensively separating and recovering tocotrienol from a raw material fluid containing the target oil constituent in an amount of several tens of percent or more. In a laboratory scale chromatographic separation targeted for research or the like, however, the economical advantages and disadvantages are not considered even when the content of oil constituent is approximately 1% or less in an aqueous raw material fluid. Although a commercial application of the method with an increased scale while the content of the oil constituent is maintained at 1% or less is possible from the technical point of view, such a method cannot be employed when the economical efficiency is taken into account because of high running costs incurred for the thermal energy, etc. For example, when a raw material fluid which is a target of the present invention is used (that is, a fluid almost entirely consisting of oil constituents), it is desirable to omit processes such as dilution and concentration in order to reduce the consumption of thermal energy. However, in the reversed-phase chromatography targeted to a raw material liquid containing a slight amount of tocopherol and which uses a polar eluent as described above, a tendency of the target oil constituent for a phase separation within the polar eluent becomes stronger as the content of the oil constituents within the raw material fluid is increased, resulting in a fatal problem wherein chromatographic separation cannot be achieved. Because of this, dilution for reducing the concentration of the target material to 1% or less is required. When the concentration is reduced, however, the consumption of thermal energy necessary for evaporating the dilution medium after separation is significantly increased. Therefore, it can be considered that conventionally, the reversed-phase chromatography has not been applied to the preparation method targeting a raw material fluid having a high content of oil constituents because there is no technique which is economically practical.

As described, as techniques related to the separation and recovery of tocotrienol which is a target of the present invention, a method disclosed in Japanese Patent Laid-Open Publication No. Hei 8-59647 is known which is a preparation method, in a commercial scale, of the tocopherol group in a less strict definition from a raw material fluid containing a large amount of tocopherol homologues (raw material fluid targeted by the present invention comprises substantially 100% oil constituents) using a normal phase chromatography. However, no effective chromatographic separation method for separating tocotrienol from tocopherol in a more strict definition (i.e., a group not including tocotrienol) treating tocopherol homologues within a raw material fluid containing tocotrienol is known.

In addition, as a technique for preparing tocopherol from a raw material fluid containing a large amount of tocopherol homologues including tocopherol and tocotrienol through a chromatographic separation, a method as disclosed in Japanese Patent Laid-Open Publication No. Hei 8-92050 which uses a normal phase chromatography is known. However, as described, in this method, it is difficult to separate α-tocopherol and α-tocotrienol, and therefore, cannot be employed for a commercial scale application of preparation of tocotrienol.

Moreover, the operation which can be performed in a laboratory scale cannot be employed because such an operation completely differs in the technical aspects from an operation for preparing (separating and recovering) tocotrienol in a commercial scale which is a target of the present invention.

The present invention was conceived in view of the conventional techniques as described above. As a result of extensive researches of a method for inexpensively separating and recovering tocotrienol from a raw material fluid containing tocopherol homologues (raw material fluid containing large amounts of tocopherol and tocotrienol) in a commercial scale and not a laboratory level, the present inventors developed and now provide a novel method.

Another advantage of the present invention is that a method is provided in which the recovery rate and the purity of tocotrienol can be maintained at a sufficiently high level while allowing application in a commercial scale.

Yet another advantage of the present invention is that a method is provided in which while the above advantages are achieved, the separation and recovery in the commercial scale can be achieved by operations not so different from the conventionally known simulated moving bed chromatographic separation and the initial equipping cost and operational cost of running can be reduced.

### Disclosure of Invention

According to one aspect of the present invention, there is provided a chromatographic method of separating and recovering tocotrienol contained in a raw material fluid by circulating the raw material fluid and an eluent through an adsorbent, wherein the raw material fluid contains tocopherol including its homologues and tocotrienol including its homologues; the adsorbent is a hydrophobic adsorbent for reversed-phase liquid chromatography having a lower affinity for the tocotrienol than the that for the tocopherol; and the eluent is a mixture of water and alcohol having a carbon number of 2 or greater..

According to another aspect of the present invention, there is provided (1) a simulated moving bed type chromatographic preparation method of separating and recovering tocotrienol from a raw material fluid thorough reversed-phase chromatography using at least four columns each filled with an adsorbent (stationary phase) having a lower polarity than a eluent (mobile phase), pipes for connecting the columns to form an overall continuous circulation flow path system which comprises cyclically and serially connected columns and pipes, fluid supplying branch pipes for each fluid branched from the pipes and each of which is provided with a valve which can open or close to allow or stop the supply of the raw material fluid or the hydrophilic eluent having a higher polarity than the adsorbent to an entrance end section of each of the columns, fluid extraction branch pipes for each fluid branched from the pipes and each of which is provided with a valve which can open or close to allow or stop the extraction of a fraction fluid enriched with the separation and recovery target constituent and extraction of a fraction fluid enriched with other constituents from the exit end section of each of the columns, and valve open and close control means for determining to which column within the circulation flow path system each of the raw material fluid and the eluent is to be supplied, determining from which column within the circulation flow path system each of the fraction fluid enriched in the separation and recovery target constituent and the fraction fluid enriched in the other constituents is to be extracted, and opening and closing each of the valves accordingly, in which the supply positions for the raw material fluid and the eluent in the circulation flow path system and the extraction positions for the fraction fluids in the circulation flow path system are determined, and the positions are respectively periodically moved by one column toward the downstream side of the fluid circulation within the circulation flow path system, wherein in order to separate and recover tocotrienol which is the target constituent from a raw material fluid containing a group of tocopherol homologues of tocopherol and tocotrienol in an amount preferably of at least several tens of percent (at least 10%) and consists substantially 100% of oil constituents, after a first through a fourth operation are performed simultaneously or sequentially, the supply positions of the fluids and extraction positions of the fluids are moved by switching the open/close state of the valves by the valve open/close control means, wherein in the first operation, each column is filled with a hydrophobic adsorbent for reversed-phase liquid chromatography having a relatively weak affinity for tocotrienol than that for tocopherol, a mixture of water and alcohol having a carbon number of 2 or greater is used as the eluent, and a fraction fluid is extracted from the exit end section of the column at a downstream position within a band enriched in tocotrienol, the fraction fluid containing the enriched constituent; in the second operation, a fraction fluid is extracted from the exit end section of the column at a downstream position within the band enriched with tocopherol and wherein substantially no tocotrienol is present, the fraction fluid containing the enriched constituent; in the third operation, a raw material fluid is supplied from the inlet section of the column at the upstream position within the band enriched with tocotrienol; and in the fourth operation, the eluent is supplied to the entrance end section of one of the columns positioned between the column at the upstream position within the band enriched with tocopherol and the column at the downstream position of the band enriched with tocotrienol.

According to yet another aspect of the present invention, there is provided (2) a simulated moving bed type chromatographic preparation method of separating and recovering tocotrienol in which are used at least four columns each filled with a hydrophilic adsorbent having a lower polarity than an eluent, pipes for connecting the columns to form an overall continuous circulation flow path system which comprises cyclically and serially connected columns and pipes, raw material fluid supplying branch pipes branched from the pipes and each of which is provided with a valve which can open or close to allow or stop the supply of the raw material fluid to an entrance end section of one of the column, an eluent supplying branch pipe branched from the pipes and which is provided with a valve which can open or close to allow or stop the supply of the eluent having a higher polarity than the adsorbent to an entrance end section of each of the columns, fluid extraction branch pipes for each fluid branched from the pipes and each of which is provided with a valve which can open and close extraction of a fraction fluid enriched in the separation and recovery target constituent or extraction of a fraction fluid enriched with other constituents from the exit end section of each of the columns, and valve open and close control means for determining to which column within the circulation flow path system each of the raw material fluid and the eluent is to be supplied, determining from which column within the circulation flow path system the fraction fluid enriched with the separation and recovery target constituent and the fraction fluid enriched in the other constituents are to be extracted, and for opening and closing each of the valves accordingly, in which a target constituent within the raw material fluid is separated and recovered through a first step for supplying, to the circulation flow path system, a raw material fluid containing three groups of constituents which can be classified as having relatively strong, intermediate, and weak affinity for the adsorbent and for extracting at least one group of constituents from among the three groups, a second step for supplying the eluent while not supplying the raw material fluid and for extracting at least one group of constituents from among the three groups, and a third step for circulating the fluid within the circulation flow path system without supplying or extracting fluid to accelerate separation of the constituents as necessary, wherein in order to separate and recover tocotrienol which is the target constituent from a raw material fluid containing a group of tocopherol homologues of a less strict definition including tocopherol and tocotrienol in an mount of preferably at least several tens of percent and consisting substantially 100% of oil constituents, an adsorbent for reversed-phase liquid chromatography with the affinity with respect to tocotrienol being the intermediate, the affinity with respect to tocopherol being the relatively strong, and the affinity with respect to the other constituents being the relatively weak is used as the adsorbent and filled into each column, and a mixture of water and alcohol having a carbon number of 2 or greater is used as the eluent; the first step is performed through at least a first operation and a third operation; the second step is performed through at least a second operation and a fourth operation without the third operation; the first operation being an operation for extracting, from the exit section of the column at a downstream position within the band enriched in tocotrienol, a fraction fluid including the enriched constituent; the second operation being an operation for simultaneously or sequentially extracting, from the exit sections of columns at a downstream position within the band enriched with tocopherol and the band enriched with constituents having a weaker affinity for the adsorbent than the tocotrienol, substantially no tocotrienol being present in these bands, the fraction fluid including the enriched constituent; the third operation being an operation for supplying the raw material fluid from the entrance end section of a column at the downstream side distanced by one or more columns away from the column from which tocopherol is extracted in the second operation; and the fourth operation being an operation for supplying the eluent from the entrance end section of one of the columns located between the column at an upstream position within the band enriched with tocopherol and the column at a downstream position within the band enriched with tocotrienol.

The term "preparation" in the above described aspects (1) and (2) of the present invention is a term used in contrast to "analysis" and is not directly related to the amount. In general, in an "analysis", the target constituent is not used for a particular application after the target constituent is separated and analyzed while in a "preparation", a target material is separated from a fluid containing a valuable material as a target constituent and is used for a particular application. The size of a preparation apparatus increases as the required amount of preparation and recovery increases.

The description "substantially 100% oil constituent" refers to a state wherein substantially no constituent is present which dissolves in water. However, in the present invention, "substantially 100% oil constituent" is not necessarily be limited to the case wherein the constituent other than oil is precisely zero and includes a case wherein a water-soluble constituent is present in an amount that does not affect the preparation operations. In the preparation of tocotrienol according to the present invention, a water-soluble constituent of approximately 1% will not interfere with the method.

The description "column at an upstream position within a band" and "column at a downstream position within a band" in general.refer respectively to a column at the upstream-most position and a column at the downstream-most position in each band when each constituent is separately enriched in a plurality of bands over an entire circulation system, but when a band comprises only one column, these description refer to said column. When a band extends over two or more unit columns, a column which is one column downstream of the upstream-most column in the band may be referred to as a "column at an upstream position within a band" and a column which is one column upstream of the downstream-most column in the band may be referred to as a "unit column at a downstream position within a band", depending on the demands for recovery rate and purity of the recovery target constituent.

The description "normal phase chromatography" refers to a method of liquid chromatography wherein a highly polar material is used as a stationary phase and a material having a lower polarity than that of the material of the stationary phase is used as a mobile phase. The description "reversed-phase chromatography" refers to a method of liquid chromatography wherein a material having a lower polarity than that of the material of the mobile phase is used as the stationary phase.

According to another aspect of the present invention, it is preferable that, (3) in the chromatographic preparation method of tocotrienol according to (2), the first step includes at least an operation to extract tocotrienol having the intermediate affinity with respect to the adsorbent and the second step includes at least an operation to extract tocopherol having the strong affinity with respect to the adsorbent and an operation to extract constituents having the weak affinity with respect to the adsorbent.

According to another aspect of the present invention, it is preferable that, (4) in the chromatographic preparation method of tocotrienol according to (2) or (3), the second step includes an operation for periodically moving the supply position of the eluent and the extraction position of the fraction fluid enriched in constituents other than tocotrienol toward the downstream side of the circulation flow path system by one column through switching of open/close state of the valves in response to a movement of the bands enriched in the constituents; and the third step is not performed.

According to another aspect of the present invention, there is provided (5) a chromatographic preparation method for separating and recovering a tocotrienol group from other oil constituents in a raw material fluid consisting substantially 100% of oil constituents and containing at least several tens of percent of tocopherol homologues including a tocopherol group and a tocotrienol group, wherein a hydrophobic adsorbent having relatively low affinity with respect to the tocotrienol group than that with respect to the tocopherol group is filled into columns; the raw material fluid and an eluent of ethanol-water are supplied to the columns; and the tocotrienol group is separated from tocopherol and recovered through reversed-phase chromatography.

According to another aspect of the present invention, it is preferable that, (6) in the chromatographic preparation method of tocotrienol according to any one of (1) - (4), the eluent consisting of alcohol and water is an ethanol-water mixture having 80% - 98.5% ethanol and remaining water or an isopropanol-water mixture having 75% - 90% isopropanol and remaining water.

In the present invention, it is important that a solution based on alcohol having a carbon number of 2 or greater and water is used as the eluent. That is, when tocotrienol family is to be prepared (isolated), in a commercial scale, by separating it from a tocopherol family of a more strict definition in a tocopherol family of a less strict definition which is an oil constituent, if a method of analysis which is targeted to a fluid under a special condition of only containing the target constituent in an amount of 1% or less is to be applied, the method cannot be realized because a phase separation occurs between the water and oil constituents. On the other hand, if a normal phase chromatography is applied which does not cause such phase separation, although it is possible to separate the tocopherol family of a less strict definition from other oil constituents, tocopherol and tocotrienol cannot be effectively separated because the properties are similar as described earlier. Therefore, conventionally, no simulated moving bed type preparation method for separating tocotrienol from tocopherol has been proposed. However, as is known to a person with ordinary skill in the art, separation and recovery of a target constituent through simulated moving bed chromatographic preparation is very advantageous as a commercial method in various aspects such as the thermal energy cost compared to distillation. After extensive researches for various combination of adsorbent, eluent, and method of normal phase or reversed-phase of chromatographic separation, the present inventors have found that preparation in a commercial scale of tocotrienol family can be realized through a reversed-phase chromatography which uses an adsorbent and an eluent of alcohol-water having predetermined properties. Such a finding is significant because it overturns the conventional common knowledge which has conventionally dictated such technique as impossible. In addition, it has been confirmed that, even when an alcohol-water based solution is used as the eluent, separation cannot effectively be achieved if methanol is used as the alcohol.

Based on these findings, it is desirable that, among various alcohol-water based solutions, a mixture of ethanol and water having 80% - 98.5% ethanol and remaining portion water, more preferably, 90% - 95% ethanol and the remaining portion water or a mixture of isopropanol and water having 75% - 90% isopropanol and the remaining portion water, more preferably, 80% - 85% isopropanol and the remaining portion water is used as the eluent in the present invention. When the alcohol concentration is lower than these ranges, the tendency for the elution property of the tocopherol and tocotrienol to weaken (tendency not to be eluted from the adsorbent) increases, resulting in problems such as extension of operation time, increases in the amount of usage of the eluent, and reduction in concentration of the extracted fraction fluid. On the other hand, when the alcohol concentration is greater than the above-described ranges, the time difference of separation of tocopherol and of tocotrienol becomes small, resulting in problems such as a tendency for insufficient separation. Here, the percentage of alcohol described above refers to the volume of alcohol before mixing. More specifically, when, for example, 90 ml of alcohol and 10 ml of water are mixed, the percentage of alcohol is defined to be 90%. The composition of the ethanol-water mixture is identical in the invention (5) described above.

According to another aspect of the present invention, it is preferable that a hydrophobic adsorbent having a low polarity as described in the following (7) is used.

(7) A chromatographic preparation method of tocotrienol according to any one of (1) - (6), wherein the adsorbent for the reversed-phase liquid chromatography is ODS-silica gel or a copolymer gel of stylene-divinyl benzene.

As the hydrophobic adsorbent, for example, silica gal in which an alkyl group is introduced is preferably used, and, in particular, ODS-silica gel is preferably used.

The "ODS-silica gel" is a known material in which octadecyl silane is introduced to a silanol group which is present on the surface of silica gel in order to adjust the polarity of the silica gel. Alternatively, a gel of a copolymer of porous styrene-divinyl benzene may also be used.

### Brief Description of Drawings

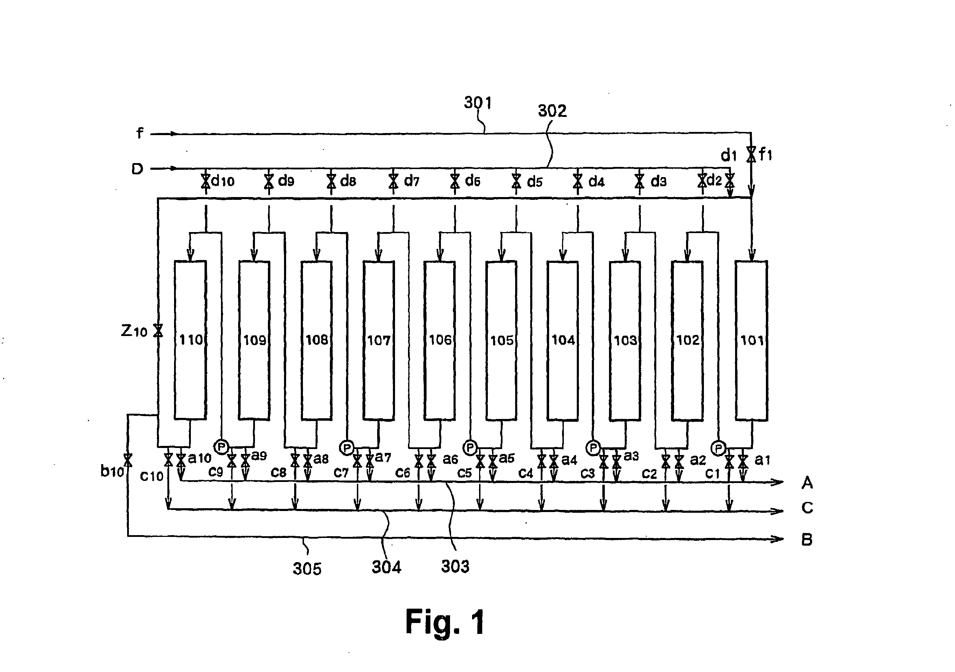

Fig. 1 is a schematic diagram showing an example structure of a simulated moving bed type chromatographic preparation apparatus used for practicing the present invention.

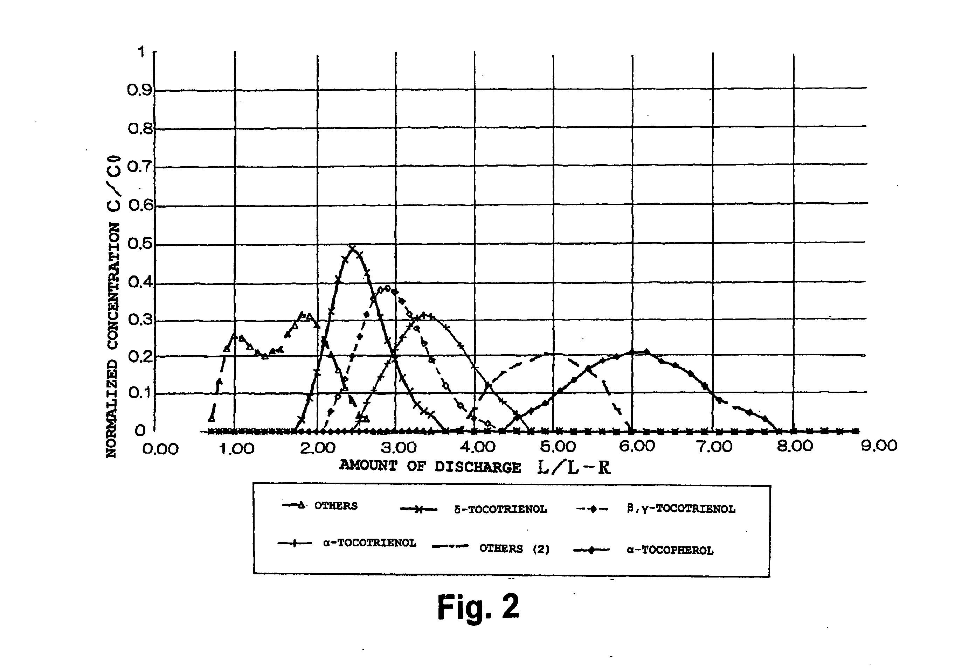

Fig. 2 is a diagram showing a distributed state, in the direction of flow of fluid, of enriched fractions of each oil constituent such as tocotrienol and tocopherol due to differences in compatibility (affinity) to the adsorbent when the preparation method according to the present invention is applied using the apparatus of Fig. 1 with a coconut oil as a raw material fluid.
Fig. 3 is a diagram showing a result of application showing distribution of enriched fractions of each oil constituent in a condition where the enriched fractions cannot be easily separated in the direction of flow of fluids due to the differences in compatibility (affinity) to the adsorbent in preparation using a raw material fluid having a large amount of tocotrienol and tocopherol through a normal phase chromatography.

### Best Mode for Carrying Out the Invention

A method of the present invention can be practiced in various systems. For example, in order to separate tocotrienol family from tocopherol family in a raw material fluid having almost no other oil constituents (including a raw material fluid which contains other oil constituents, but which may be considered as substantially two-constituents because the affinity of the other oil constituents with respect to the adsorbent is similar to that of the tocopherol family in a more strict definition), a typical simulated moving bed type method for separation of two constituents may be employed wherein positional relationships among the positions for supplying the raw material fluid and for supplying an eluent and the extraction positions for a fraction enriched in tocotrienol family and for a fraction enriched in tocopherol family are set to predetermined positional relationships, and all positions are periodically moved to the downstream side by one column. In the above description, a "constituent" refers to a constituent that can be separated by a difference in affinity with respect to the adsorbent, and does not mean a difference in chemical composition or the like (similarly in the following descriptions).

In order to separate three constituents from a raw material fluid containing the above-mentioned two constituents and a third oil constituent having a different affinity than the two constituents with respect to the adsorbent, a method can be employed as disclosed in Japanese Patent Laid-Open Publication No. Hei 4-227804, wherein the "simulated moving bed method for separation of two constituents" performed in the above second step refers to an operation wherein, in a cyclical serial circulation flow path of a plurality of columns filled with an adsorbent, while liquid is circulated within the system by a pump or the like, an eluent is supplied from an upstream position of a band enriched with a predetermined constituent, an enriched fraction is extracted at a downstream position of the band, and the positions are sequentially moved to the downstream of the circulation flow in response to the movement of the adsorption band.

The second step as described above can also be performed by a pre-step of circulating liquid within a circulation flow path while supplying an eluent and no raw material fluid to the circulation flow path and extracting fractions enriched with the constituent (or a plurality of constituents) which remains from the first step at one or more downstream positions of the band and a post-step for extracting each enriched fraction from downstream positions in the adsorption band of each of the constituents which are distributed in the order of increasing affinity while supplying an eluent and circulating liquid within the circulation flow path, and periodically and sequentially switching the supplying position of the eluent and the extraction positions for enriched fractions in response to the movement of the adsorption band by a column to the downstream side.

In addition, when the constituent extracted from one extraction point comprises a plurality of compositions, these compositions can be extracted as one group without any distinction among the compositions, or, alternatively, it is also possible, for example, to extract the plurality of compositions in separate times (preparation in time series) . More specifically, for compositions having different affinity, by extracting while dividing the extraction timing into three times of initial, intermediate, and final, it is possible to separate compositions in an enriched fraction for which the distribution is further divided.

The method of the present invention can also be practiced in various forms wherein other operations are added to the operations described above. For example, it is possible to supply an eluent to the system of the circulation flow path system in the first step. In this configuration, it is preferable that the supply position of the eluent is provided on an upstream position within an adsorption band in which the constituent having the strongest affinity with respect to the adsorbent is distributed or on a position further upstream of such position. It is also possible to extract, from the system, simultaneously other constituents (for example, constituents having weak affinity) divided into adsorption bands in the first step.

An apparatus for practicing the method described above is not limited, and can be constructed by, for example, forming a cyclical serial circulation flow path using a plurality of columns which are filled with an adsorbent, the number of which being selected from 4 - 20, and more preferably, 8 - 14, providing a fluid supply inlet and a fluid withdrawal exit, both of which can be switched to open and close, between each column in the circulation flow path, and providing fluid circulation means such as a pump for circulating fluid in the circulation flow path.

The shutdown in the operation of the first step can be performed by controlling the supply process and extraction process of fluid such that substantially no flow of fluid occurs in particular positions. In order to facilitate this process, it is also possible to provide a shutdown valve which can be opened and closed along the circulation flow (in general, at one point or two points) . That is, the first step can be performed by providing a shutdown valve at one or more sections in the route in the circulation flow path sequentially connecting a plurality of columns, supplying a raw material fluid from a position immediately downstream of the shutdown valve while the shutdown valve is closed, and extracting tocotrienol at a position immediately upstream of the shutdown valve while supplying an eluent to the column at a predetermined position in order to extract a constituent having an intermediate affinity (tocotrienol).

The raw material fluid of tocotrienol (raw material fluid of tocopherol of a less strict definition including tocotrienol and tocopherol) used in the method of the present invention is not limited as long as the fluid contains tocotrienol family, and examples include coconut oil (palm oil), rice bran oil, soybean oil, and so on.

In addition, when the amount of eluent is not an issue, the present invention can be practiced by a three-zone method described in, for example, Japanese Patent Laid-Open Publication No. Sho 62-91205 wherein the eluent recovery band in the second step is omitted.

### Examples

Fig. 1 shows an example apparatus of the present invention used in the preparation method for separating and recovering tocotrienol from a raw material fluid containing tocotrienol at a high concentration (50% or greater in the raw material fluid). In Fig. 1, reference numerals 101 - 110 represent a group of columns which are filled with an ODS silica gel adsorbent (hereinafter simply referred to as "columns"). The exit end section of each column is connected to an entrance end section of the next column with a pipe so that the columns are connected in an endless cyclically connected circulation system.

In the example structure, on partway of the pipe between the columns 110 and 101, a shutdown valve (cutting valve) z10 is provided so as to enable forced shutdown of the flow within the circulation system as necessary.

Reference numeral 301 represents a supply pipe (supply line) for a raw material fluid f which is connected to the column 101 through a valve f1 which can be opened and closed, so as to enable supply of the raw material fluid from the entrance end section of the column 101. Reference numeral 302 represents a supply pipe (supply line) for eluent which is connected to the columns 101 -110 respectively through supply valves d1 - d10, so as to enable supply of eluent from the entrance end section of each of the columns 101 - 110.

Reference numeral 303 represents an extraction pipe for a weakly adsorbed constituent and is connected to the columns 101 - 110 respectively through extraction valves a1 - a10 so as to enable extraction of the weakly adsorbed constituent (fraction enriched with other constituents) from the columns 101 - 110. Reference numeral 304 represents an extraction pipe for a strongly adsorbed constituent and is connected to the exit end sections of the columns 101 - 110 respectively through extraction valves c1 - c10 so as to enable a fluid of strongly adsorbed constituent (fraction enriched with α-tocopherol) from the exit end section of each of the columns 101 - 110. Reference numeral 305 represents an extraction pipe for a fluid of intermediately adsorbed constituent (fraction enriched with tocotrienol) and is connected to the column 110 through an extraction valve b10 so as to enable extraction of the intermediately adsorbed constituent therefrom.

This apparatus is operated such that in the first step, the shutdown valve (cutting valve) z10 is closed so that the flow in the circulation system is shut down (cut) at the position of the shutdown valve z10 and, at the same time, a raw material fluid is supplied from the entrance end section of the column 101 through the f1 valve, an eluent is supplied to the entrance end section of the column 106 through the d6 valve which is opened, a fluid of weakly adsorbed constituent (fraction enriched with other constituents) is extracted from the exit end section of the column 103 through the a3 valve which is opened, and a fluid enriched with intermediately adsorbed constituent (fraction enriched with tocotrienol) is extracted from the exit end section of the column 110 through the b10 valve which is opened.

In the example structure, the first step through the tenth step form one cycle and the cycles are repeated. Therefore, in the beginning of the first step of one cycle, the constituents fractioned from the raw material fluid supplied to the column 101 in the previous cycle still remains in the columns.

In other words, the fluid of the intermediately adsorbed constituent (fraction enriched with tocotrienol) supplied to the column 101 in the first step in the previous cycle and separated through circulation of the fluid during the second to the tenth steps in the previous cycle is isolated in the column 110. In the first step, therefore, while a raw material fluid is supplied to the column 101, tocotrienol is extracted from the column 110. Similarly, at the beginning of the first step, the fluid of the weakly adsorbed constituents (fraction enriched with other constituents) are present in the column 103 from the previous cycle, and thus, the other constituents are extracted from the column 103.

In the first step, it is also possible to introduce a portion of the eluent to the column 106.

Then, in the second step, a circulation flow is introduced in the columns by opening the shutdown valve z10. At the same time, an eluent is supplied to the entrance end section of the column 107 through the d7 valve which is opened, fluid of a weakly adsorbed constituent (fraction enriched with other constituents) is extracted from the exit end section of the column 104 through the a4 valve which is opened, and fluid of a strongly adsorbed constituent (fraction enriched with α-tocopherol) is extracted from the exit end section of the column 108 through the c8 valve which is opened. In this first step, no raw material fluid is supplied to the column 101. Although a raw material fluid may be supplied, such supply may adversely affect separation of tocotrienol.

The fluid of strongly adsorbed constituent in the raw material fluid supplied to the column 101 in the first step of the previous cycle which has the highest tendency to be adsorbed to the adsorbent is positioned in a column which is upstream of the fluid or the intermediately adsorbed constituent. Because of this, in the second step, a fluid of a strongly adsorbed constituent is extracted from the column 108 to which the fluid of the strongly adsorbed constituent is adsorbed. During this process, the eluent is supplied to the column 107 which is immediately upstream of the adsorption band of the fluid of the strongly adsorbed constituent to accelerate movement of the fluid of the strongly adsorbed constituent caused by the eluent. For the same reason, the eluent is supplied to the column 106 in the first step.

In this manner, three divisions having different fluid flow rates are formed in the circulation system. More specifically, in the initial state of the second step in Fig. 1, with the position where the eluent is supplied (entrance end section of the column 107) as a starting point, from the upstream side to the downstream side, a group of columns 107 and 108 forms an elution operation band (first band) , a group of columns 109 - 104 forms a separation and purification operation band (second hand), and a group of columns 105 and 106 forms an eluent recovery band (third band).

In the elution operation band (first band), the fluid of the strongly adsorbed constituent is transported by the eluent, in the separation and purification operation band (second band), new separation and purification process are performed by supplying the circulation fluid to a newly supplied raw material fluid, and in the eluent recovery band (third band), a fluid in which all of the fluids of strongly, intermediately, and weakly adsorbed constituents are removed (i.e., substantially the eluent) is circulated.

From the second step to the tenth step, the apparatus is operated under typical conditions for a simulated moving bed type apparatus except that no raw material fluid is supplied. In other words, by periodically transitioning the supply position of eluent and the extraction positions for each enriched fraction fluid by one column along the direction of the fluid flow, a simulated flow of adsorbent is created and the other constituents and α-tocopherol are extracted. The apparatus is operated such that in the subsequent first step, the remaining tocotrienol is separated and recovered. Therefore, in the third step which is the subsequent step, a group of columns 108 and 109 forms the elution operation band (first band), a group of columns 110 - 105 forms the separation and purification operation band (second band), and a group of columns 106 and 107 forms the eluent recovery band (third band). Although the total number of columns is 10 and the number of columns in each zone is set in a particular configuration in the example structure shown in Fig. 1, it should be understood that the present invention is not limited to such a configuration.

It is also possible to extract the fluid of the strongly adsorbed constituent from the column 107 in the first step.

In this manner, according to the example structure described above, the fluid of the intermediately adsorbed constituent is extracted from the column 110 which is the farthest away column from the column 101 to which a raw material fluid is supplied. Therefore, it is possible to separate the fraction containing tocotrienol which is the target in a state wherein separation of constituents are advanced to a final stage. On the other hand, with regard to the fluid of the weakly adsorbed constituent, the fluid of the weakly adsorbed constituent from the previous cycle remains in the column 103 when the raw material fluid is supplied to the column 101 at the first step, and the fluid of the weakly adsorbed constituent in the current step is also mixed in the subsequent steps.

### Test Example 1

An apparatus shown in Fig. 1 was employed as a simulated moving bed type chromatographic separation device for multi-constituent separation wherein 10 columns each having an inner diameter of 2.2 cm and a length of 150 cm were connected cyclically and in series, An ODS silica gel (FS1830 manufactured by Organo Corp.; ODS silica gel having an average particle size of 100 microns) was used as the adsorbent, a solution of CH₃CH₂OH/H₂O = 90/10 was used as the eluent, and the temperature within the column was maintained at 30 °C. A separation and purification process of a raw material fluid in which a raw material of tocotrienol from coconut palm having a composition as described in the following Table 1 was dissolved in the eluent in a concentration of 20 wt/wt% was performed under operation conditions with the ratio between the liquid flow rate and the flow rate of the solid phase in each band being U1/Us = 3.5, U2/Us = 0.88, and U3/Us = 0.5 and the supply amount of raw material fluid being 3 (L/D). In this process, the processing capability of solid state amount of raw material fluid was 0.49 kg/D.

**[Table 1]**

| | OTHERS | α-TOCOTRI | β, γ₋TOCOTRI | δ-TOCOTRI | OTHERS (2) | α-TOCOPHE |
|---|---|---|---|---|---|---|
| COMPOSITION (w/w%) | 13.6 | 13.4 | 23..8 | 24.3 | 8.6 | 16.3 |

In Table 1, "tocotri" represents "tocotrienol" and "tocophe" represents "tocopherol" (similar abbreviation is used in the subsequent Tables).

In the first test example, because the differences in the affinities with respect to the adsorbent have the relationships, [others < δ-tocotrienol, γ-tocotrienol, (β-tocotrienol, and α-tocotrienol <α-tocopherol) high purity tocotrienol was extracted from the pipe 305 in the first step.

The following Table 2 shows a result (compositional structure) when a multi-constituent simulated moving bed type chromatographic separation apparatus was operated under these conditions.

From this, high purity tocotrienol was produced at a rate of 0.265 kg/D.

**[Table 2]**

| | | OTHERS | δ-TOCOTRI | β, γ-TOCOTRI | α-TOCOTRI | OTHERS (2) | α-TOCOPHE |
|---|---|---|---|---|---|---|---|
| 303 | ^{PURITY(w/w%)} | 99.8 | 0.1 | 0.1 | 0.0 | 0.0 | 0.1 |
| _{A} | RECOVERY RATE(w/w%) | 95.0 | 0.1 | 0.1 | 0.0 | 0.0 | 0.1 |
| 304 | PURITY(w/w%) | 1.2 | 24.6 | 40.0 | 34.0 | 0.2 | 0.0 |
| B | RECOVERY RATE(w/w%) | 4.8 | 98.0 | 89.9 | 75.0 | 1.0 | 0.1 |
| 305 | PURITY(w/w%) | 0.1 | 0.7 | 7.1 | 18.1 | 25.4 | 48.6 |
| C | RECOVERY RATE(w/w%) | 0.2 | 1.8 | 10.0 | 25.0 | 99.0 | 99.8 |

### Comparative Example 1

Using the same operation conditions as in the test example 1 except that an eluent of CH₃OH/H₂O = 95/5 was used, that U1/Us = 3.3, U2/Us = 1.5, and U3/Us = 0.6, and that the supply amount of raw material fluid was 3.71 (L/D), separation and purification of tocotrienol was performed. Table 3 shows the results. In this case, although the raw material dissolved in the eluent, the concentration which allowed stable operation was 1% and the processing capability of solid state amount of the raw material was 0.03 kg/D.

In the comparative example 1, high purity tocotrienol was prepared only in an amount of 0.017 kg/D.

**[Table 3]**

| | | OTHERS | δ-TOCOTRI | β, γ-TOCOTRI | α-TOCOTRI | OTHERS(2) | α-TOCOPHE |
|---|---|---|---|---|---|---|---|
| 303 | PURITY(w/w%) | 99.2 | 0.2 | 0.2 | 0.0 | 0.0 | 0.4 |
| A | RECOVERY RATE(w/w%) | 96.0 | 0.2 | 0.1 | 0.0 | 0.0 | 0.3 |
| 304 | PURITY(w/w%) | 1.0 | 23.8 | 40.8 | 32.1 | 2.4 | 0.0 |
| B | RECOVERY RATE(w/w%) | 4.0 | 99.8 | 95.4 | 74.4 | 15.6 | 0.1 |
| 305 | PURITY(w/w%) | 0.0 | 0.0 | 2.7 | 20.4 | 23.8 | 53.1 |
| C | RECOVERY RATE(w/w%) | 0.0 | 0.0 | 3.5 | 25.6 | 84.4 | 99.6 |

### Comparative Example 2

In order to improve the processing capability from the comparative example 1, the apparatus was operated under conditions wherein the supply amount of raw material was increased (load of 3.85 (L/D) which is twice the comparative example 1) . The processing capability of solid state amount of the raw material in this configuration was 0.062 kg/D. Table 4 shows the results of this comparative example.

In this comparative example 2, high-purity tocotrienol could not be obtained.

**[Table 4]**

| | | OTHERS | δ-TOCOTRI | β, γ-TOCOTRI | α-TOCOTRI | OTHERS(2) | α-TOCOPHE |
|---|---|---|---|---|---|---|---|
| 303 | PURITY(w/w%) | 63.8 | 24.6 | 0.0 | 0.0 | 0.1 | 11.6 |
| A | RECOVERY RATE(w/w%) | 98.0 | 37.8 | 0.0 | 0.0 | 0.2 | 14.8 |
| 304 | PURITY(w/w%) | 0.5 | 14.0 | 39.2 | 29.8 | 8.6 | 8.0 |
| B | RECOVERY RATE (w/w%) | 2.0 | 62.2 | 98.3 | 73.1 | 59.9 | 29.5 |
| 305 | PURITY(w/w%) | 0.0 | 0.0 | 2.1 | 33.7 | 17.7 | 46.6 |
| C | RECOVERY RATE (w/w%) | 0.0 | 0.0 | 1.7 | 26.9 | 40.0 | 55.7 |

### Industrial Applicability

As described above, the present invention provides a novel method for allowing an inexpensive separation and recovery of tocotrienol from a raw material fluid containing tocopherol family (a raw material fluid containing tocopherol and tocotrienol in a large amount) in a commercial scale rather than in a laboratory scale. One advantage of the present invention is that while commercial scale application is enabled which cannot be expected in the conventional art, a simulated moving bed type preparation technique can be provided in which the recovery rate and purity of tocotrienol can be maintained at a sufficiently high level.

In addition to the advantage described above, an advantage can be obtained by the present invention that the commercial scale separation and recovery can be realized in a method in which the operation itself is similar to the conventionally known simulated moving bed type chromatographic separation, resulting in lower initial investment cost and lower running cost.

In particular, although the eluent is limited to the use of an alcohol-water based solutions having a carbon number of 2 or greater, by using a method of a reversed-phase chromatography with such a eluent, it is possible to more efficiently prepare tocotrienol family from a raw material fluid containing tocopherol family and tocotrienol family in a commercial scale, and, therefore, another advantage can be obtained by the present invention that preparation of tocotrienol which is very much economical in terms of the thermal energy cost or the like compared to the typical conventional commercial methods such as distillation can be achieved . The present invention is very meaningful as the present invention overturns the conventional common knowledge. The present inventors have also confirmed that even when an alcohol-water based solution as described above is used as the eluent, if the alcohol is methanol, efficient separation cannot be achieved.

In the present invention, an ODS-silica gel or the like is preferably used as an adsorbent for the reversed-phase liquid chromatography. The present invention therefore has another advantage that the use of such an adsorbent, which has been widely used in analysis techniques but hardly in industrial preparation techniques employing reversed-phase chromatography, is extended.

## Claims

1. A chromatographic method for separating and recovering tocotrienol contained in a raw material fluid by circulating the raw material fluid and an eluent through an adsorbent, wherein
the raw material fluid contains tocopherol including its homologues and tocotrienol including its homologues;
the adsorbent is a hydrophobic adsorbent for reversed-phase liquid chromatography having a lower affinity for the tocotrienol than that for the tocopherol; and
the eluent is a mixture of water and alcohol having a carbon number of 2 or greater.

2. A chromatographic method for separating and recovering of tocotrienol according to claim 1, wherein
at least one of the raw material fluid and the eluent can be introduced not only from the introduction side of the adsorbent but also from an intermediate position of the adsorbent; and
a treated fluid can be discharged not only from the discharging side of the adsorbent but also from an intermediate position of the adsorbent.

3. A chromatographic method for separating and recovering tocotrienol according to claim 2, wherein
a fraction of tocotrienol is discharged from a band of the adsorbent enriched with tocotrienol;
a fraction of tocopherol is discharged from a band of the adsorbent enriched with tocopherol;
the raw material fluid is introduced at a position which is upstream of the band of the adsorbent enriched with tocotrienol; and
the eluent is introduced at a position which is upstream of the band of the adsorbent enriched with tocopherol and downstream of the band of the adsorbent enriched with tocotrienol.

4. A chromatographic method for separating and recovering tocotrienol according to claim 3, wherein
the adsorbent is filled in a divided form into a plurality of endlessly and cyclically connected columns;
the raw material fluid or the eluent can be introduced into each column; and
the treated fluid can be discharged from each column.

5. A chromatographic method for separating and recovering tocotrienol according to claim 1, wherein
the raw material fluid contains other constituents in addition to the tocotrienol and the tocopherol; and
the adsorbent has the highest affinity for the tocopherol, the next highest affinity for the tocotrienol, and the lowest affinity for the other constituents.

6. A chromatographic method for separating and recovering tocotrienol according to claim 5, wherein
at least one of the raw material fluid and the eluent can be introduced not only from the introduction side of the adsorbent but also from an intermediate position of the adsorbent, and
a treated fluid can be discharged not only from the discharging side of the adsorbent but also from an intermediate position of the adsorbent.

7. A chromatographic method for separating and recovering tocotrienol according to claim 6, wherein
a fraction of tocotrienol is discharged from a band of the adsorbent enriched with tocotrienol;
a fraction of tocopherol is discharged from a band of the adsorbent enriched with tocopherol;
the other constituents are discharged from a band enriched with the other constituents;
the raw material fluid is introduced at a position which is upstream of the band of the adsorbent enriched with the other constituents; and
the eluent is introduced at a position which is upstream of the band of the adsorbent enriched with tocopherol and downstream of the band enriched with the other constituents.

8. A chromatographic method for separating and recovering tocotrienol according to claim 7, wherein
the adsorbent is filled in a divided form into a plurality of endlessly and cyclically connected columns;
the raw material fluid or the eluent can be introduced to each column; and
the treated fluid can be discharged from each column.

9. A chromatographic method for separating and recovering tocotrienol according to any one of claims 1 - 8, wherein
the raw material fluid comprises substantially 100% oil constituents.

10. A chromatographic method for separating and recovering tocotrienol according to any one of claims 1 - 9, wherein
the treated fluid discharged from the discharging side of the adsorbent can be circulated to the introduction side.

11. A simulated moving bed type chromatographic method for separating and recovering tocotrienol from a raw material fluid thorough a reversed-phase chromatography using at least four columns each filled with an adsorbent having a lower polarity than an eluent, pipes for connecting the columns to form an overall continuous circulation flow path system which comprises cyclically and serially connected columns and pipes, fluid supplying branch pipes for each fluid branched from the pipes and each of which is provided with a valve which can open and close to allow and stop the supply of the raw material fluid or the eluent having a higher polarity than the adsorbent to an entrance end section of each of the columns, fluid extraction branch pipes for each fluid branched from the pipes and each of which is provided with a valve which can open and close to allow or stop extraction of a fraction fluid enriched with the separation and recovery target constituent and extraction of a fraction fluid enriched with other constituents from the exit end section of each of the columns, and valve open and close control means for determining to which column within the circulation flow path system each of the raw material fluid and the eluent is to be supplied, determining from which column within the circulation flow path system each of the fraction fluid enriched with the separation and recovery target constituent and the fraction fluid enriched with the other constituents is to be extracted, and opening and closing each of the valves accordingly, in which the supply positions for the raw material fluid and the eluent in the circulation flow path system and the extraction positions for the fraction fluids in the circulation flow path system are determined, and the positions are respectively periodically moved by one column toward the downstream side of the fluid circulation within the circulation flow path system, wherein
in order to separate and recover tocotrienol which is the target constituent from a raw material fluid containing a group of tocopherol homologues of tocopherol and tocotrienol and consisting substantially of 100% of oil constituents, after a first operation through a fourth operation are performed simultaneously or in sequence, the supply positions of the fluids and extraction positions of the fluids are moved by switching the open/close state of the valves by the valve open/close control means, wherein
in the first operation, each column is filled with a hydrophobic adsorbent for reversed-phase liquid chromatography having a relatively low affinity for tocotrienol than that for tocopherol, a mixture of water and alcohol having a carbon number of 2 or greater is used as the eluent, and a fraction fluid is extracted from the exit end section of the column at a downstream position within a band enriched with tocotrienol, the fraction fluid containing said enriched constituent;
in the second operation, a fraction fluid is extracted from the exit end section of the column at a downstream position within the band enriched with tocopherol and wherein substantially no tocotrienol is present, the fraction fluid containing said enriched constituent;
in the third operation, a raw material fluid is supplied from the entrance end section of the column at the upstream position within the band enriched with tocotrienol; and
in the fourth operation, the eluent is supplied to the entrance end section of one of the columns positioned between the column at the upstream position within the band enriched with tocopherol and the column at the downstream position of the band enriched with tocotrienol.

12. A simulated moving bed type chromatographic method for separating and recovering tocotrienol which uses at least four columns each filled with an adsorbent having a lower polarity than an eluent, pipes for connecting the columns to form an endless and overall continuous circulation flow path system which comprises cyclically and serially connected columns and pipes, raw material fluid supplying branch pipes branched from the pipes and each of which is provided with a valve which can open or close to allow or stop the supply of the raw material fluid to an entrance end section of one of the columns, eluent supplying branch pipes branched from the pipes, each of which is provided with a valve which can open and close to allow and stop the supply of the eluent having a higher polarity than the adsorbent to an entrance end section of each of the columns, fluid extraction branch pipes for each fluid branched from the pipes, each of which is provided with a valve which can open and close to allow or stop the extraction of a fraction fluid enriched with the separation and recovery target constituent or extraction of a fraction fluid enriched with other constituents from the exit end section of each of the columns, and valve open and close control means for determining to which column within the circulation flow path system each of the raw material fluid and the eluent is to be supplied, determining from which column within the circulation flow path system the fraction fluid enriched with the separation and recovery target constituent and the fraction fluid enriched with the other constituents are to be extracted, and for opening and closing each of the valves accordingly, in which a target constituent within the raw material fluid is separated and recovered through a first step for supplying, to the circulation flow path system, a raw material fluid containing three groups of constituents which can be classified as having relatively strong, intermediate, and weak affinity for the adsorbent and for extracting at least one group of constituents from among the three groups, a second step for supplying the eluent while not supplying the raw material fluid and for extracting at least one group of constituents from among the three groups, and a third step for circulating the fluid within the circulation flow path system without supplying or extracting fluid to accelerate separation of the constituents as necessary, wherein
in order to separate and recover tocotrienol which is the target constituent from a raw material fluid containing a group of tocopherol homologues including tocopherol and tocotrienol and consisting substantially 100% of oil constituents, an adsorbent for reversed-phase liquid chromatography with the affinity for tocotrienol being the intermediate, the affinity for tocopherol being the relatively strong, and the affinity for the other constituents being the relatively weak is used as the adsorbent and filled into each column, and a mixture of water and alcohol having a carbon number of 2 or greater is used as the eluent;
the first step is performed through at least the undermentioned first operation and third operation;
the second step is performed through at least the undermentioned second operation and fourth operation without the third operation;
the first operation being an operation for extracting, from the exit end section of the column at a downstream position within the band enriched with tocotrienol, a fraction fluid including said enriched constituent;
the second operation being an operation for simultaneously or sequentially extracting, from the exit end sections each of which columns at a downstream position within the band enriched with tocopherol and the band enriched with constituents having a lower affinity for the adsorbent than tocotrienol, substantially no tocotrienol being present in these bands, the fraction fluid including said constituent(s);
the third operation being an operation for supplying the raw material fluid from the entrance end section of a column at the downstream side distanced by one or more columns away from the column from which tocopherol having a high affinity for the adsorbent is extracted in the second operation; and
the fourth operation being an operation for supplying the eluent from the entrance end section of one of the columns located between the column at an upstream position within the band enriched with tocopherol and the column at a downstream position within the band enriched with said other constituents.

13. A chromatographic method for separating and recovering tocotrienol according to claim 12, wherein
the first step includes at least an operation to extract tocotrienol having the intermediate affinity for the adsorbent and
the second step includes at least an operation to extract tocopherol having the strong affinity for the adsorbent and an operation to extract constituents having the weak affinity for the adsorbent.

14. A chromatographic method for separating and recovering tocotrienol according to claim 12 or 13, wherein
the second step includes an operation for periodically moving the supply position of the eluent and the extraction position of the fraction fluid enriched with constituents other than tocotrienol toward the downstream side of the circulation flow path system by one column at a time through switching of open/close state of the valves in response to the movement of the bands enriched with respective constituents; and
the third step is not taken.

15. A chromatographic method for separating and recovering a tocotrienol group from other oil constituents in a raw material fluid consisting substantially 100% of oil constituents and containing at least several tens of percent of tocopherol homologues including a tocopherol group and a tocotrienol group, wherein
a hydrophobic adsorbent having relatively low affinity for the tocotrienol group than that for the tocopherol group is filled into columns;
the raw material fluid and an eluent of ethanol-water are supplied to the columns; and
the tocotrienol group is separated from tocopherol and recovered through reversed-phase chromatography.

16. A chromatographic method of separating and recovering tocotrienol according to any one of claims 1 - 15, wherein
the eluent made of alcohol and water is an ethanol-water mixture having 80% - 98.5% ethanol and remaining water or an isopropanol-water mixture having 75% - 90% isopropanol and remaining water.

17. A chromatographic method of separating and recovering tocotrienol according to any one of claims 1 - 16, wherein
the adsorbent for the reversed-phase liquid chromatography is ODS-silica gel or a copolymer gel of stylene-divinyl benzene.
